Europäisches Patentamt

European Patent Office

Office européen des brevets

(11) Veröffentlichungsnummer: 0 496 366 A1

## EUROPÄISCHE PATENTANMELDUNG

(21) Anmeldenummer: 92100987.4

(51) Int. Cl.⁵: **A61M 15/00, H01R 13/447**

(22) Anmeldetag: 22.01.92

(30) Priorität: 23.01.91 DE 4101825

(43) Veröffentlichungstag der Anmeldung:
29.07.92 Patentblatt 92/31

(84) Benannte Vertragsstaaten:
AT BE CH DE DK ES FR GB GR IT LI LU MC
NL PT SE

(71) Anmelder: **Hane, Heinz**
**Erzgebirgstrasse 7**
**W-8900 Augsburg(DE)**
Anmelder: **Kuss, Peter**
**Bannzaunweg 5**
**W-8024 Deisenhofen(DE)**
Anmelder: **Kromesch, Elke**
**Mannheimer Strasse 97**
**W-6806 Viernheim(DE)**

(72) Erfinder: **Hane, Heinz**
**Erzgebirgstrasse 7**
**W-8900 Augsburg(DE)**
Erfinder: **Kuss, Peter**
**Bannzaunweg 5**
**W-8024 Deisenhofen(DE)**
Erfinder: **Kromesch, Elke**
**Mannheimer Strasse 97**
**W-6806 Viernheim(DE)**

(74) Vertreter: **Munk, Ludwig, Dipl.-Ing.**
**Patentanwalt Prinzregentenstrasse 1**
**W-8900 Augsburg(DE)**

(54) Inhalationsgerät.

(57) Um sicherzustellen, daß ein Inhalationsgerät mit einem eine Flüssigkeits-Reaktionskammer aufnehmenden Grundkörper, einen Deckel zum gasdichten Abschluß zumindest der Flüssigkeits-Reaktionskammer sowie einem Anschluß für eine flexible, zu einer Atemmaske führende Leitung nicht nur zur Inhalation von Sauerstoff, sondern auch von Dampf mit ätherischen Ölen verwendet werden kann, ist im Flüssigkeits-Reaktionsbehälter (5) eine elektrische Heizeinrichtung (13) mit einer Steckbuchse für einen Stecker (17) einer elektrischen Zuleitung (18) angeordnet und der Grundkörper (1) vor der Steckbuchse mit einer Durchtrittsöffnung (44) versehen, die mittels eines Verschlußelements (22) abdeckbar ist, wobei der Deckel (2) in drei Positionen auf den Grundkörper (1) aufsetzbar ist sowie einen Blockieransatz (25) aufweist, der in der ersten Position des Verschlußelement (22) in der Schließstellung arretiert und in der zweiten Position zur Öffnung freigibt.

FIG 1

Die Erfindung betrifft ein Inhalationsgerät mit einem mindestens eine als Flüssigkeits-Reaktionskammer ausgebildete Flüssigkeitskammer aufnehmenden Grundkörper, einem Deckel zum gasdichten Abschluß zumindest der Flüssigkeits-Reaktionskammer sowie einem Anschluß für eine flexible, zu einer Atemmaske führende Leitung.

Derartige Geräte werden zur Inhalation von Sauerstoff benutzt. Hierzu werden in die mit Wasser partiell gefüllte Flüssigkeits-Reaktionskammer Pulver eingebracht, die durch eine chemische Reaktion zu einer Sauerstoffentwicklung führen. Der in der Flüssigkeits-Reaktionskammer oberhalb des Flüssigkeitsspiegels anfallende, unter Druck stehende Sauerstoff tritt dann durch einen Überströmkanal und eine weitere, Wasser enthaltende Flüssigkeitskammer in einen Sammelraum, an den die zur Atemmaske führende flexible Leitung anschließbar ist.

Der Erfindung liegt die Aufgabe zugrunde, ein derartiges Gerät so weiterzuentwickeln, daß es bei einfachem Aufbau und bedienungssicherer Handhabung nicht nur zur Inhalation von Sauerstoff, sondern auch von Dampf mit ätherischen Ölen verwendet werden kann.

Erfindungsgemäß wird dies dadurch erreicht, daß im Flüssigkeitsreaktionsbehälter eine elektrische Heizeinrichtung mit einer Steckbuchse für einen Stecker einer elektrischen Zuleitung angeordnet ist, der Grundkörper vor der Steckbuchse eine Durchtrittsöffnung aufweist, die mittels eines Verschlußelementes abdeckbar ist, und der Deckel in zwei Positionen auf den Grundkörper aufsetzbar ist, sowie einen Blockieransatz aufweist, der in der ersten Position das Verschlußelement in der Schließstellung arretiert, in der zweiten Position dagegen zur Öffnung freigibt.

Mit einem derartigen Inhalationsgerät kann zum einen bei in Schließstellung befindlichem Verschlußelement in üblicher Weise eine Sauerstoffinhalation folgen. Eine versehentliche, unerwünschte Aufheizung des in der Flüssigkeits-Reaktionskammer befindlichen Wassers ist aber ausgeschlossen. Zum anderen kann bei in Öffnungsstellung befindlichem Verschlußelement und angeschlossener elektrischer Zuleitung eine Verdampfung des in der Flüssigkeits-Reaktionskammer befindlichen Wassers mit einem Zusatz ätherischer Öle erfolgen.

Vorteilhaft ist das Verschlußelement als Schieber ausgebildet. Eine besonders einfache und bedienungssichere Ausgestaltung ergibt sich dabei, wenn der Schieber in Aufsetzrichtung des Deckels bewegbar gelagert ist und der Blockieransatz in der ersten Position des Deckels entgegen der Öffnungsrichtung des Schiebers bis zur Anlage an dem in Schließstellung befindlichen Schieber in dessen Öffnungsweg einführbar ist.

Eine weitere Vereinfachung des Aufbaus des Grundkörpers ergibt sich, wenn der Schieber in einem Führungskanal des Grundkörpers angeordnet ist und der Grundkörper einen weiteren Führungskanal zur Aufnahme des Blockieransatzes in der zweiten Position des Deckels aufweist.

Zur Fixierung des Deckels am Grundkörper sind vorteilhaft am Deckel federnde Rastklauen und am Grundkörper Rastansätze angeordnet. Hiermit kann der Deckel unter Vermeidung beweglich gelagerter Teile am Grundkörper festgelegt werden.

Um dem Benutzer eine einfache, betriebssichere Handhabung des Gerätes zu ermöglichen, ist der Deckel mit einer Anzeigemarke versehen, während der Grundkörper zwei wechselweise damit zusammenwirkende Markierungen trägt.

Vorteilhaft ist die Heizeinrichtung als eine in ein am Grundkörper befestigtes Rohr eingesetzte, eine Heizwendel und eine keramische Umhüllung aufweisende Heizpatrone ausgebildet. Dabei ist das Rohr am einen Ende abgedichtet verschlossen und am anderen Ende mit der Steckbuchse verbunden. Hierdurch ergibt sich ein konstruktiv einfacher Aufbau der Heizeinrichtung, die sich auch leicht montieren läßt.

Um in einfacher Weise einen gasdichten Abschluß zwischen Deckel und Grundkörper zu erzielen, weist der Deckel alle seitlichen Begrendungswände der Flüssigkeits-Reaktionskammer sowie der zusätzlichen Flüssigkeitskammer in aufgesetztem Zustand beiderseits übergreifende Stege auf. Dabei ist zwischen zwei, eine Begrenzungswand übergreifenden Stegen ein elastisches Dichtungsmaterial eingebracht.

Ein Ausführungsbeispiel der Erfindung ist anhand der Zeichnung beschrieben. In dieser zeigt:

Figur 1     ein erfindungsgemäßes Inhalationsgerät mit dem in der einen Position aufgesetzten Deckel in einem senkrechten Längsschnitt und

Figur 2     das Inhalationsgerät nach Figur 1 mit dem in der anderen Position aufgesetzten Deckel, teils im Längsschnitt, teils in Ansicht.

Das dargestellte Inhalationsgerät weist einen insgesamt mit 1 bezeichneten Grundkörper und einen insgesamt mit 2 bezeichneten Deckel auf. Der Grundkörper 1 nimmt eine durch seitliche Begtenzungswände 3, 4 gebildete Flüssigkeits-Reaktionskammer 5 mit einem Überströmkanal 6 auf. Dabei ist der Überströmkanal 6 vom unteren Teil der Flüssigkeits-Reaktionskammer5 durch eine Wandung 7 abgeteilt. Der Überströmkanal 6 führt zu einem mit Bohrungen 8 versehenen Wandteil 9, der gleichzeitig einen Teil des Bodens einer zweiten Flüssigkeitskammer 10 bildet. Die Flüssigkeitskammer 10 ist seitlich durch die Begrenzungswand 3 sowie eine weitere Begrenzungswand 11 begrenzt. Die Flüssigkeits-Reaktionskammer 5 und die zweite Flüssigkeitskammer 10 sind durch weitere, nicht näher dargestellte, in Längsrichtung des Grundkörpers 1 verlaufende Wände begrenzt, deren Höhe, wie die gestrichelte Linie 12 zeigt, gleich der Höhe der Begrenzungswände 3, 4 und 11 ist. Diese Wände können teilweise durchsichtig ausgebildet sein, wodurch sich Sichtfenster der bei 5a bzw. 10a angedeuteten Art ergeben. Hierdurch ist es möglich, daß der Benutzer die Höhe des Flüssigkeitsspiegels und/oder die Flüssigkeit durchziehende Gasperlen etc. erkennen kann.

Über dem Boden der Flüssigkeits-Reaktionskammer 5 ist eine insgesamt mit 13 bezeichnete Heizeinrichtung angeordnet. Die Heizeinrichtung 13 umfaßt ein in die Flüssigkeits-Reaktionskammer 5 hineinragendes Rohr 14, das zweckmäßig aus Aluminium hergestellt ist. Das Rohr 14 ist an seinem in die Flüssigkeits-Reaktionskammer 5 hineinragenden Ende mittels eines Bodens 15 abgedichtet verschlossen. Das Rohr 14 ist mit seinem offenen Ende in eine Anschlußhülse 16 eingesetzt. Die Anschlußhülse 16 durchsetzt eine Öffnung in der Begrenzungswand 4 und ist fest mit dieser verbindbar. Das Rohr 14 nimmt eine nicht näher dargestelle, eine Heizwendel mit einer keramischen Umhüllung aufweisende Heizpatrone auf. In der Anschlußhülse 16 ist eine ebenfalls nicht näher dargestellte Steckbuchse angeordnet, die zum Anschluß eines Steckers 17 einer elektrischen Zuleitung 18 dient.

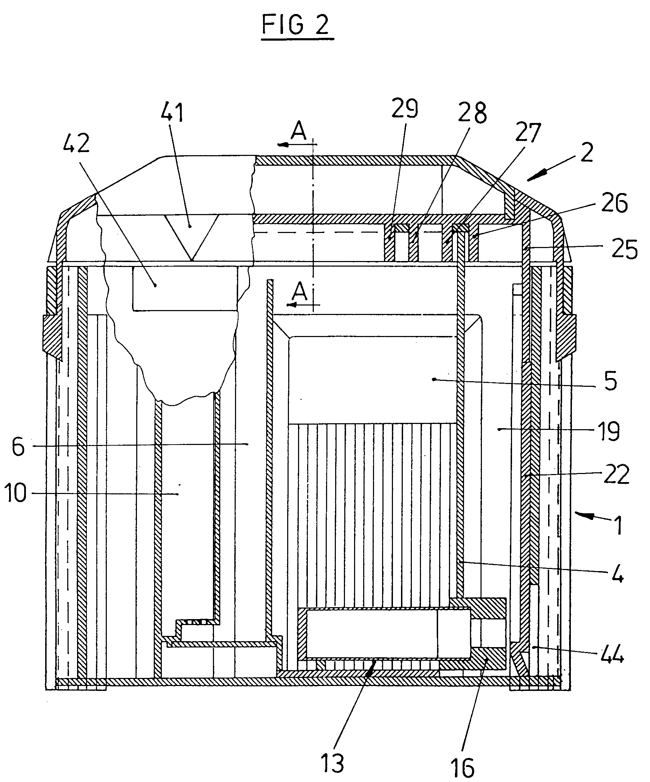

Die Anschlußhülse 16 ragt in einen Führungskanal 19 hinein, der einerseits durch die Begrenzungswand 4 und andererseits durch eine Wand 20 begrenzt ist. Zwischen der Wand 20 und mit dieser verbundenen Führungsleisten 21 ist ein als Verschlußelement dienender Schieber 22 in bzw. entgegen der Richtung des Pfeiles a so bewegbar geführt, daß er eine Durchtrittsöffnung 44 in der Wand 20 freigeben oder abdecken kann. Der Abstand zwischen der Begrenzungswand 4 und der Wand 20 ist dabei so bemessen, daß der Schieber 22 vor das äußere Ende der Anschlußhülse 16 geführt werden kann, wie Figur 2 zeigt. In dieser Stellung deckt der Schieber 22 die Durchtrittsöffnung 44 zu der in der Anschlußhülse 16 vorgesehenen Steckbuchse ab, so daß der Stecker 17 nicht angesetzt werden kann. Außen vor der Begrenzungswand 11 ist eine Wand 23 vorgesehen, die zusammen mit der Begrenzungswand 11 einen weiteren Führungskanal 24 begrenzt. Die beiden Führungskanäle 19 und 24 sind in ihren Abmessungen gleich ausgebildet.

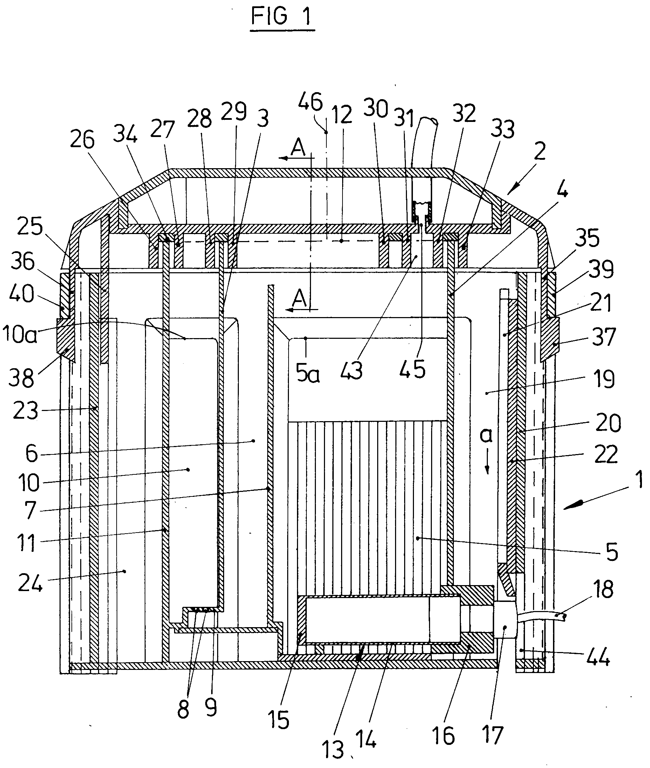

Am Deckel 2 ist ein Blockieransatz 25 vorgesehen, der in der einen, in Figur 1 dargestellten Aufsetzposition des Deckels 2 in den Führungskanal 24 hineinragt. In dieser Aufsetzposition des Deckels 2 kann daher der Schieber 22 in eine Lage überführt werden, in der ein zugang mit dem Stecker 17 zur Anschlußhülse 16 möglich ist. In der anderen, in Figur 2 wiedergegebenen Aufsetzposition des Deckels 2 ragt dagegen der Blockieransatz 25 in den Verschiebeweg des Schiebers 22 hinein, so daß der Schieber 22 in der Lage festgelegt ist, in der er einen Zutritt zur Anschlußhülse 16 verhindert.

An der Unterseite des Deckels 2 sind weiterhin paarweise Stege 26 bis 33 angebracht. Zwischen den beiden Stegen eines Stegpaares, z.B. 26, 27, ist ein elastisches Dichtmaterial 34 eingebracht. Da der Abstand der Begrenzungswände 4 und 11 von den Außenseiten des Gehäuses 1 gleich bemessen und der Deckel 2 symmetrisch zu einer Mittelquerebene A-A ausgebildet ist, kann der Deckel 2 zum einen so auf den Grundkörper 1 aufgesetzt werden, daß die Stege 26, 27 die Begrenzungswand 11, die Stege 32, 33 die Begrenzungswand 4 und die Stege 28, 29 die Begrenzungswand 3 übergreifen. Durch/Drehung des Deckels 2 um 180° läßt sich dieser jedoch auch so aufsetzen, wie Figur 2 zeigt, daß die Stege 26, 27 die Begrenzungswand 4 übergreifen. Dann umfassen die Stege 32, 33 die Begrenzungswand 11.

Am Deckel 2 sind weiterhin mittels federnder Laschen 35, 36 zwei Rastklauen 37, 38 angebracht. Die Rastklauen 37, 38 greifen beim Aufsetzen des Deckels 2 hinter feste Rastansätze 39, 40 des Grundkörpers 2 und legen dadurch den Deckel 2 am Grundkörper 1 fest.

Zur Erleichterung der Handhabung des Gerätes ist, wie Figur 2 zeigt, an der Außenseite des Deckels 2 eine Marke 41 angebracht. Die Marke 41 wirkt in der in Figur 2 wiedergegebenen Position mit einer am Grundkörper 2 vorgesehenen Markierung 42 zusammen. In der in Figur 1 wiedergegebenen Lage des Deckels 2 wird diese Marke mit einer weiteren, an der gegenüberliegenden Längsseite des Gehäuses 1 angegebenen Markierung zusammen.

In der Position der Teile gemäß Figur 1 wird die Flüssigkeits-Reaktionskammer 5 mit Wasser und ätherischen Ölen partiell angefüllt. Anschließend wird der Schieber 22 in die Öffnungsstellung überführt und der Stecker 17 der elektrischen Zuleitung 18 angesteckt. Dabei hält der Stecker 17 den Schieber 22 in der Öffnungsstellung. Die in der Flüssigkeits-Reaktionskammer 5 enthaltenen Flüssigkeiten verdampfen und treten in einen zwischen den Stegen 31, 32 vorgesehenen Sammelraum 43, von dem aus sie über eine als Wandausnehmung ausgebildete, obere Abflußöffnung 45 und einen dieser zugeordneten Anschluß sowie eine hieran ansetzbare, flexible Leitung zur Atemmaske gelangen. Zur Durchführung einer Sauerstoff-Inhalation werden die Flüssigkeits-Reaktionskammer 5, der Überströmkanal 6 und die zweite Flüssigkeitskammer 10 mit Wasser gefüllt. Anschließend werden in den Flüssigkeits-Reaktionsraum 5 die zur Sauerstoffbildung erforderlichen chemischen Mittel eingefüllt. Dann wird der Deckel, wie in Figur 2 gezeigt, aufgesetzt. Hierbei wird der Schieber 22 in seiner Schließstellung mittels des Blockieransatzes 25 festgelegt, so daß nicht versehentlich die Heizeinrichtung 13 aktiviert werden kann. Der entstehende Sauerstoff tritt über die Überströmleitung 6 und die zweite Flüssigkeitskammer 10 wiederum in den Sammelraum 43 zwischen den Stegen 31, 32, von wo aus er zur Atemmaske gelangt. Der über die Überströmleitung 6 und die zweite Flüssigkeitskammer 10 zum Sammelraum 43 in Form von Perlen überströmende Sauerstoff kann über das Sichtfenster 10a von außen beobachtet werden. Es ist daher möglich, die Wirkzeit der eingebrachten chemischen Mittel, also die zeitliche Dauer der Sauerstofferzeugung von außen festzustellen. Außerdem ermöglicht die hier vorgesehene U-förmige Sauerstoffüberleitung zur Abflußöffnung auch eine Reinigung des Sauerstoffs sowie eine langsame, sehr sparsame Abgabe des Sauerstoffs.

Es wäre selbstverständlich auch denkbar, den Sauerstoff wie den Dampf ohne Umlenkung abzunehmen. Hierzu könnte anstelle der Öffnung 45 einfach eine im Bereich zwischen den Stegen 29, 30 plazierte Öffnung vorgesehen sein, wie durch die Mittellinie 46 angedeutet ist.

Die Erfindung ist nicht auf das dargestellte Ausführungsbeispiel beschränkt. So könnte beispielsweise das Verschlußelement auch als schwenkbare Klappe ausgebildet sein. Auch könnten die Marke am Grundkörper 1 und die Markierungen am Deckel 2 angebracht sein.

**Patentansprüche**

1. Inhalationsgerät mit einem mindestens eine als Flüssigkeits-Reaktionskammer (5) ausgebildete Flüssigkeitskammer aufnehmenden Grundkörper (1), einem Deckel (2) zum gasdichten Abschluß zumindest der Flüssigkeits-Reaktionskammer (5) sowie einem Anschluß (45) für eine flexible, zu einer Atemmaske führende Leitung, dadurch gekennzeichnet, daß in der Flüssigkeits-Reaktionskammer (5) eine elektrische Heizeinrichtung (13) mit einer Steckbuchse für einen Stecker (17) einer elektrischen Zuleitung (18) angeordnet ist, der Grundkörper (1) vor der Steckbuchse eine Durchtrittsöffnung (44) aufweist, die mittels eines Verschlußelementes (22) abdeckbar ist, der Deckel (2) in zwei Positionen auf den Grundkörper (1) aufsetzbar ist sowie einen Blockieransatz (25) aufweist, der in der ersten Position das Verschlußelement (22) in der Schließstellung arretiert, in der zweiten Position dagegen zur Öffnung freigibt.

2. Inhalationsgerät nach Anspruch 1, dadurch gekennzeichnet, daß das Verschlußelement als Schieber (22) ausgebildet ist.

3. Inhalationsgerät nach Anspruch 2, dadurch gekennzeichnet, daß der Schieber (22) in Aufsetzrichtung des Deckels (2) bewegbar gelagert ist und der Blockieransatz (25) in der ersten Aufsetzposition des Deckels (2) entgegen der Öffnungsrichtung des Schiebers (22) bis zur Anlage an den in Schließstellung befindlichen Schieber (22) in dessen Öffnungsweg einführbar ist.

4. Inhalationsgerät nach Anspruch 1 oder 2, dadurch gekennzeichnet, daß der Schieber (22) in einem Führungskanal (19) des Grundkörpers (1) angeordnet ist und der Grundkörper (1) einen weiteren Führungskanal (24) zur Aufnahme des Blockieransatzes (25) in der zweiten Position des Deckels (2) aufweist.

5. Inhalationsgerät nach einem der vorhergehenden Ansprüche, dadurch gekennzeichnet, daß zur Fixierung des Deckels (2) am Grundkörper (1) am Deckel (2) federnde Rastklauen (37, 38) und am Grundkörper (1) Rastansätze (39, 40) vorgesehen sind.

6. Inhalationsgerät nach einem der vorhergehenden Ansprüche, dadurch gekennzeichnet, daß der Deckel (2) mit einer Anzeigemarke (41) versehen ist und der Grundkörper (1) zwei wechselweise damit zusammenwirkende Markierungen (42) trägt.

7. Inhalationsgerät nach einem der vorhergehenden Ansprüche, dadurch gekennzeichnet, daß die Heizeinrichtung (13) über dem Bodenbereich der Flüssigkeits-Reaktionskammer (5) angeordnet ist.

8. Inhalationsgerät nach einem der vorhergehenden Ansprüche, dadurch gekennzeichnet, daß die Heizeinrichtung (13) als eine in ein am Grundkörper (1) befestigtes Rohr (14) eingesetzte, eine Heizwendel mit einer keramischen Umhüllung aufweisende Heizpatrone ausgebildet ist und das Rohr (14) am einen Ende abgedichtet verschlossen und am anderen Ende mit der Steckbuchse verbunden ist.

9. Inhalationsgerät nach einem der vorhergehenden Ansprüche mit einer zweiten Flüssigkeitskammer (10), dadurch gekennzeichnet, daß der Deckel (2) beide Flüssigkeitskammern (5, 10) gasdicht verschließt, die durch einen Überströmkanal (6), der vorzugsweise durch ein mit Bohrungen (8) versehenes Wandteil (9) begrenzt ist, miteinander verbunden sind und daß der mit einer Abströmöffnung (45) versehene Anschluß für die Atemmaske so plaziert ist, daß er in jeder der beiden Stellungen des Deckels (2) mit einer anderen Flüssigkeitskammer (5 bzw. 10) kommuniziert.

10. Inhalationsgerät nach einem der vorhergehenden Ansprüche, dadurch gekennzeichnet, daß der Deckel (2) alle Begrenzungswände (3, 4, 11) der Flüssigkeits-Reaktionskammer (5) und der zusätzlichen Flüssigkeitskammer (10) im aufgesetzten Zustand beiderseits übergreifende Stege (26 bis 33) aufweist und zwischen zwei, eine Begrenzungswand übergreifenden Stegen ein elastisches Dichtungsmaterial (34) eingebracht ist.

## FIG 1

# FIG 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A | FR-A-2 095 264 (HEWLETT-PACKARD COMPANY) <br> * Anspruch 1; Abbildungen 1-4 * <br> --- | 1 | A61M15/00 <br> H01R13/447 |
| A | FR-A-1 027 718 (ANTOINE) <br> * Seite 1, linke Spalte, Zeile 21 - rechte Spalte, Zeile 5 * <br> --- | 1 | |
| A | FR-A-2 571 262 (MERCIER ET AL.) <br> * Zusammenfassung; Abbildungen * <br> * Seite 6, Zeile 1 - Zeile 12 * <br> --- | 1 | |
| A | DE-A-3 639 836 (BECHTER) <br> * Zusammenfassung; Ansprüche; Abbildungen * <br> ----- | 1 | |

**RECHERCHIERTE SACHGEBIETE (Int. Cl.5 )**

A61M
H01R

Der vorliegende Recherchenbericht wurde für alle Patentansprüche erstellt

| Recherchenort | Abschlußdatum der Recherche | Prüfer |
|---|---|---|
| DEN HAAG | 17 MAERZ 1992 | ZEINSTRA H. |